(19) Europäisches Patentamt
European Patent Office
Office européen des brevets

(11) EP 4 011 863 A1

(12) **EUROPEAN PATENT APPLICATION**
published in accordance with Art. 153(4) EPC

(43) Date of publication:
**15.06.2022 Bulletin 2022/24**

(21) Application number: **20850955.4**

(22) Date of filing: **04.08.2020**

(51) International Patent Classification (IPC):
***C07C 255/23*** *(2006.01)* ***C09J 4/04*** *(2006.01)*

(52) Cooperative Patent Classification (CPC):
**C07C 255/23**

(86) International application number:
**PCT/JP2020/029905**

(87) International publication number:
**WO 2021/025036 (11.02.2021 Gazette 2021/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.08.2019 JP 2019146572**

(71) Applicant: **Toagosei Co., Ltd.**
**Minato-ku**
**Tokyo 105-8419 (JP)**

(72) Inventors:
- **KONDO, Kei**
**Nagoya-shi, Aichi 455-0026 (JP)**
- **ISSHIKI, Erika**
**Nagoya-shi, Aichi 455-0026 (JP)**
- **ISHIZAKI, Kenichi**
**Nagoya-shi, Aichi 455-0026 (JP)**
- **OKAZAKI, Eiichi**
**Nagoya-shi, Aichi 455-0026 (JP)**

(74) Representative: **Rüger Abel Patentanwälte PartGmbB**
**Patentanwälte**
**Webergasse 3**
**73728 Esslingen a. N. (DE)**

(54) **2-CYANOACRYLATE COMPOUND AND ADHESIVE COMPOSITION**

(57) A2-cyanoacrylate compound represented by Formula (1) and an adhesive composition containing the 2-cyanoacrylate compound represented by Formula (1). In Formula (1), $R^1$ represents a divalent linking group having 1 to 20 carbon atoms, $R^2$ and $R^3$ each independently represent an alkylene group having 2 to 4 carbon atoms, and $R^4$ represents an alkyl group having 1 to 20 carbon atoms.

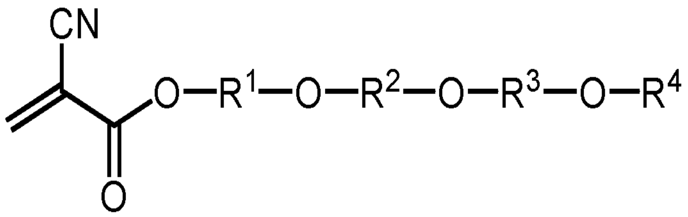

(1)

EP 4 011 863 A1

## Description

The present invention relates to a novel 2-cyanoacrylate compound and a novel adhesive composition.

In an adhesive composition containing a 2-cyanoacrylate compound, polymerization is initiated by weak anions such as slight moisture attached to a surface of an adherend due to specific anionic polymerizability of the 2-cyanoacrylate compound as a main component, and various materials can be firmly bonded in a short time. Therefore, as a so-called instant adhesive, it is used in a wide range of fields such as industrial uses, medical uses, and household uses.

Examples of techniques related to conventional 2-cyanoacrylate compounds or methods for producing the same, or conventional adhesive compositions containing a 2-cyanoacrylate compound include those described in Patent Documents 1 to 5.

Patent Documents 1 and 2 describe conventional methods for manufacturing a 2-cyanoacrylate.

Patent Document 3 describes a novel 2-cyanoacrylate represented by the following structural formula.

$$CH_2=C(CN)COOR_1OR_2$$

Provided that, in the formula, $R_1$ represents a lower alkylene group, and $R_2$ represents a halogenated lower alkyl group.

Patent Document 4 describes a cyanoacrylate adhesive composition having improved water solubility after being cured, which is obtained by blending a water-soluble polyoxyalkylene glycol-based solvent and a water-soluble surfactant in a cyanoacrylate.

Patent Document 5 describes a fast-setting adhesive composition containing 0.1 to 10 parts by weight of a compound having, on average, 5 to 30 ethylene oxide residues and two or more (meth)acryloyl groups in the molecule, and 0.05 to 5 parts by weight of a monohydric alcohol having 5 or less carbon atoms based on 100 parts by weight of an $\alpha$-cyanoacrylate monomer.

Patent Document 1: WO 2017/135184
Patent Document 2: Japanese National-Phase Publication (JP-A) No. 2017-514796
Patent Document 3: Japanese Patent Application Laid-Open (JP-A) No. H8-283225
Patent Document 4: Japanese Patent Application Laid-Open (JP-A) No. 2000-73015
Patent Document 5: Japanese Patent Application Laid-Open (JP-A) No. H8-188747

An object of the present invention is to provide a novel 2-cyanoacrylate compound.

Another object of the present invention is to provide an adhesive composition containing the 2-cyanoacrylate compound.

Means for solving the problem include the following aspects.

<1> A 2-cyanoacrylate compound represented by the following Formula (1).

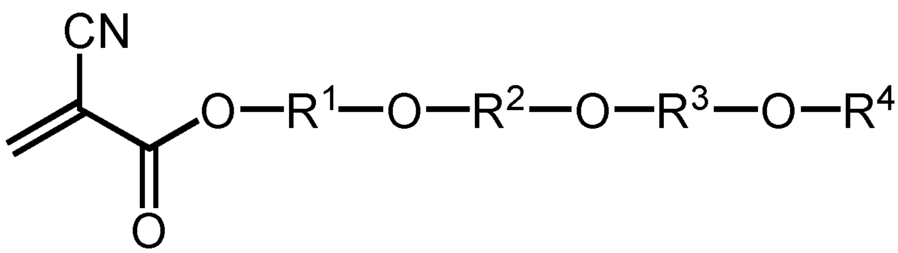

(1)

In Formula (1), $R^1$ represents a divalent linking group having 1 to 20 carbon atoms, $R^2$ and $R^3$ each independently represent an alkylene group having 2 to 4 carbon atoms, and $R^4$ represents an alkyl group having 1 to 20 carbon atoms.

<2> The 2-cyanoacrylate compound according to <1>, wherein $R^1$ is an alkylene group having 1 to 20 carbon atoms.

<3> The 2-cyanoacrylate compound according to <1> or <2>, wherein $R^1$ is an alkylene group having 1 to 8 carbon atoms.

<4> The 2-cyanoacrylate compound according to any one of <1> to <3>, wherein $R^2$ and $R^3$ are each independently an ethylene group, a 1,2-propylene group, or a 1,3-propylene group.

<5> The 2-cyanoacrylate compound according to any one of <1> to <4>, wherein $R^4$ is an alkyl group having 1 to 6 carbon atoms.

<6> The 2-cyanoacrylate compound according to any one of <1> to <5>, wherein $R^4$ is an alkyl group having 1 to

4 carbon atoms.
<7> An adhesive composition containing the 2-cyanoacrylate compound according to any one of <1> to <6>.

The present invention can provide a novel 2-cyanoacrylate compound.
Further, the present invention can provide an adhesive composition containing the 2-cyanoacrylate compound.

Fig. 1 shows a $^1$H-NMR spectrum of a 2-cyanoacrylate compound obtained in Example 1.
Fig. 2 shows a $^{13}$C-NMR spectrum of the 2-cyanoacrylate compound obtained in Example 1.
Fig. 3 shows a $^1$H-NMR spectrum of a 2-cyanoacrylate compound obtained in Example 2.
Fig. 4 shows a $^{13}$C-NMR spectrum of the 2-cyanoacrylate compound obtained in Example 2.
Fig. 5 shows a $^1$H-NMR spectrum of a 2-cyanoacrylate compound obtained in Example 3.
Fig. 6 shows a $^{13}$C-NMR spectrum of the 2-cyanoacrylate compound obtained in Example 3.
Fig. 7 shows a $^1$H-NMR spectrum of a 2-cyanoacrylate compound obtained in Example 4.
Fig. 8 shows a $^{13}$C-NMR spectrum of the 2-cyanoacrylate compound obtained in Example 4.
Fig. 9 shows a $^1$H-NMR spectrum of a 2-cyanoacrylate compound obtained in Example 5.
Fig. 10 shows a $^{13}$C-NMR spectrum of the 2-cyanoacrylate compound obtained in Example 5.

The description of the components described below may be made based on representative embodiments of the present invention, but the present invention is not limited to such embodiments. As used herein, the term "to" is used to mean that numerical values indicated before and after the term "to" are included as a lower limit value and an upper limit value.
In the numerical ranges described in stages herein, the upper limit value or the lower limit value described in one numerical range may be replaced with the upper limit value or the lower limit value of any other numerical range described in stages. In addition, in the numerical ranges described herein, the upper limit values or the lower limit values of the numerical ranges may be replaced with values shown in Examples.
In the present invention, the terms "mass%" and "weight%" have the same meaning, and the terms "parts by mass" and "parts by weight" have the same meaning.
In the present invention, a combination of two or more preferred embodiments is a more preferred embodiment.
Hereinafter, contents of the present invention will be described in detail.

(2-Cyanoacrylate compound represented by Formula (1))

The 2-cyanoacrylate compound of the present invention is a 2-cyanoacrylate compound represented by the following Formula (1).

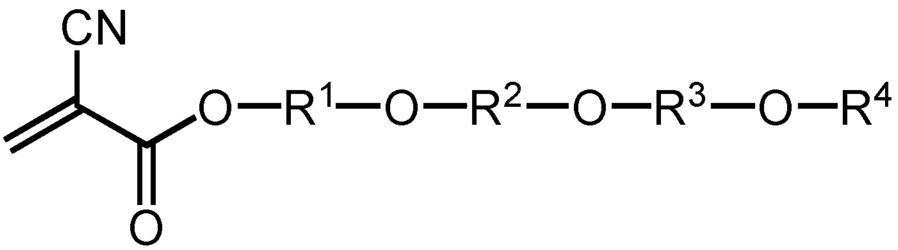

( 1 )

In Formula (1), $R^1$ represents a divalent linking group having 1 to 20 carbon atoms, $R^2$ and $R^3$ each independently represent an alkylene group having 2 to 4 carbon atoms, and $R^4$ represents an alkyl group having 1 to 20 carbon atoms.
The 2-cyanoacrylate compound represented by Formula (1) above is a novel compound, is a highly reactive compound not only as a monomer but also capable of anionic polymerization by an anionic polymerization initiating species such as water, and can be suitably used as an adhesive component of an adhesive composition.
$R^1$ in Formula (1) may be linear, branched, or cyclic.
A number of carbons (also referred to as "number of carbon atoms") of $R^1$ in Formula (1) is more preferably 1 to 8, yet more preferably 1 to 4, and particularly preferably 2 or 3 from the viewpoint of reactivity, stability, and adhesion of the resulting cured product to a substrate having polarity.
The alkylene groups in $R^2$ and $R^3$ of Formula (1) may each be linear, branched, or cyclic.
$R^2$ and $R^3$ in Formula (1) are each independently preferably an ethylene group, a 1,2-propylene group, or a 1,3-propylene group from the viewpoint of stability and reactivity of the compound.
A number of carbon atoms of $R^4$ in Formula (1) is preferably 1 to 10, more preferably 1 to 6, and yet more preferably 1 to 4 from the viewpoint of reactivity, stability, and adhesion of the resulting cured product to a substrate

having polarity.

The alkyl group in $R^4$ may be linear, branched, or cyclic.

Further, $R^4$ may have a substituent. Examples of the substituent include an alkyl group, an aryl group, a halogen atom, a cyano group, an alkoxycarbonyl group, an aryloxycarbonyl group, an acyl group, and an acyloxy group. Among them, an alkyl group or a halogen atom is preferable, and an alkyl group is more preferable.

Furthermore, $R^4$ in Formula (1) is preferably a linear or branched alkyl group having 1 to 10 carbon atoms, more preferably a linear or branched alkyl group having 1 to 6 carbon atoms, yet more preferably a linear or branched alkyl group having 1 to 4 carbon atoms, and particularly preferably a methyl group, from the viewpoint of reactivity, stability, and adhesion of the resulting cured product to a substrate having polarity.

Among them, the 2-cyanoacrylate compound represented by Formula (1) is preferably a 2-cyanoacrylate compound represented by the following Formula (2) or Formula (3), more preferably a 2-cyanoacrylate compound represented by the following Formula (3), and particularly preferably a 2-cyanoacrylate compound represented by the following Formula (4), from the viewpoint of reactivity, stability, and adhesion of the resulting cured product to a substrate having polarity. In addition, a 2-cyanoacrylate compound represented by the following Formula (2) is particularly preferable from the viewpoint of easy water-dismantlability of the resulting cured product.

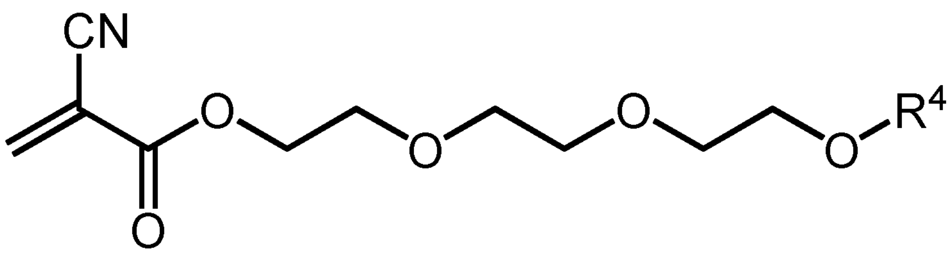

(2)

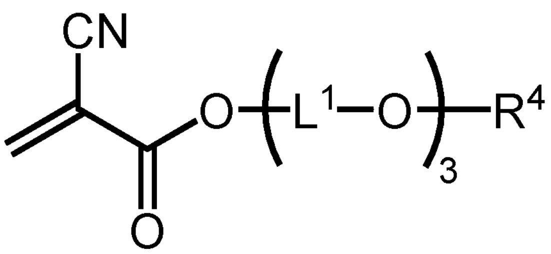

(3)

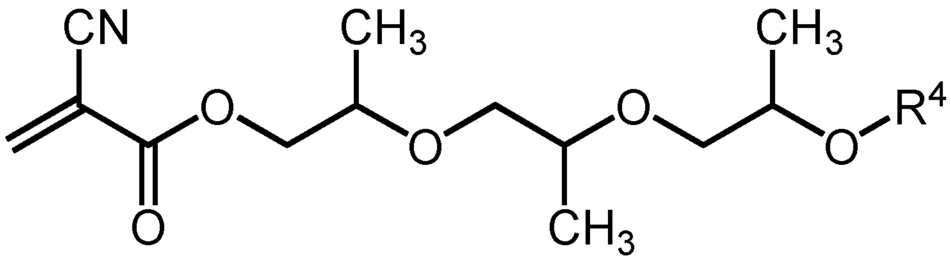

(4)

In Formulae (2) to (4), $R^4$ represents an alkyl group having 1 to 20 carbon atoms, and $L^1$'s each independently represent $-CH_2CH(CH_3)-$ or $-CH(CH_3)CH_2-$.

$R^4$ in Formulae (2) to (4) is preferably a linear or branched alkyl group having 1 to 10 carbon atoms, more preferably a linear or branched alkyl group having 1 to 6 carbon atoms, yet more preferably a linear or branched alkyl group having 1 to 4 carbon atoms, and particularly preferably a methyl group, from the viewpoint of reactivity, stability, and adhesion of the resulting cured product to a substrate having polarity.

A method for synthesizing the 2-cyanoacrylate compound represented by Formula (1) is not particularly limited, and the 2-cyanoacrylate compound can be synthesized by combining known synthesis means.

Examples of the method include a method in which a cyanoacetic acid ester compound and formaldehyde or paraformaldehyde are subjected to a dehydration condensation reaction in the presence of a base catalyst to obtain a condensation polymer, which is then heated and depolymerized to give a 2-cyanoacrylate compound having an ethylenically unsaturated bond at an $\alpha$-position thereof. In addition, the cyanoacetic acid ester compound can be easily produced, for example, by reacting an alcohol compound corresponding to cyanoacetic acid in the presence of an acid catalyst to perform esterification.

As another method, for example, a 2-cyanoacrylate compound, which is different from the 2-cyanoacrylate compound represented by Formula (1), such as ethyl -2 cyanoacrylate, is reacted with a diene compound to form an adduct, the adduct is induced to a carboxylic acid of the adduct by alkaline hydrolysis, and the adduct is reacted with a corresponding alcohol compound in the presence of an acid catalyst to perform esterification, and then the esterified product is thermally decomposed at an appropriate temperature, whereby the 2-cyanoacrylate compound represented by Formula (1) can be obtained. Also, the 2-cyanoacrylate compound represented by Formula (1) can be obtained by subjecting an adduct of a 2-cyanoacrylate compound and a diene compound and a corresponding alcohol compound to a transesterification reaction in the presence of a Lewis acid catalyst and thermally decomposing the obtained ester compound.

As still another method, the 2-cyanoacrylate compound represented by Formula (1) can also be obtained by reacting cyanoacrylic acid chloride with a corresponding alcohol compound.

The 2-cyanoacrylate compound represented by Formula (1) can be suitably used for an adhesive composition, has 2 or 3 alkyleneoxy structures, and therefore has a higher polarity than that of conventional 2-cyanoacrylate compounds, and thus can be particularly suitably used for an easily water-dismantlable adhesive composition.

The easily water-dismantlable adhesive composition is an adhesive composition excellent in peelability or removability with water after being cured.

When the adhesive composition is used as an easily water-dismantlable adhesive composition, it preferably further contains a water-soluble compound which will be described below, from the viewpoint of easy water-dismantlability.

When used in an adhesive composition, the 2-cyanoacrylate compound represented by Formula (1) has a content of preferably 1 mass% or more and 100 mass% or less, more preferably 2 mass% or more and 99 mass% or less, yet more preferably 5 mass% or more and 98 mass% or less, particularly preferably 10 mass% or more and 95 mass% or less, and most preferably 15 mass% or more and 95 mass% or less, with respect to the total mass of the adhesive composition, from the viewpoint of easy water-dismantlability, adhesion to a substrate having polarity, and curability.

When used in an adhesive composition, particularly in an easily water-dismantlable adhesive composition, the adhesive composition preferably further contains a water-soluble compound.

In the present invention, the term "water-soluble compound" means a compound that is admixed with water in an arbitrary mixing ratio to form a solution, or has a solubility in water (25°C) of 1 g/100 g or more.

A solubility parameter (SP value) of the water-soluble compound is preferably 8.0 $(cal/cm^3)^{0.5}$ or more and 23.4 $(cal/cm^3)^{0.5}$ or less, more preferably 8.3 $(cal/cm^3)^{0.5}$ or more and 15.0 $(cal/cm^3)^{0.5}$ or less, yet more preferably 9.0 $(cal/cm^3)^{0.5}$ or more and 14.0 $(cal/cm^3)^{0.5}$ or less, and particularly preferably 8.0 $(cal/cm^3)^{0.5}$ or more and 12.0 $(cal/cm^3)^{0.5}$ or less, from the viewpoint of easy water-dismantlability.

The solubility parameter (SP value) in the present invention is a value calculated by the calculation method described in "Polymer Engineering and Science" 14(2), 147 (1974) authored by R.F. Fedors. Specifically, the calculation method shown in Formula (3) is used. Note that 2.0455 $(cal/cm^3)^{0.5}$ = 1 $MPa^{0.5}$.

$$\delta = \sqrt{\frac{\sum \Delta E_{vap}}{\sum V}} \qquad (3)$$

δ: SP value $((cal/cm^3)^{1/2})$
ΔEvap: molar evaporation heat (cal/mol) of each atomic group
V: molar volume of each atomic group ($cm^3$/mol)

In addition, in a case where two or more kinds are used in combination, calculation is performed by the following equation.

$$(\text{SP value of mixture})^2 = (\text{volume fraction of component 1}) \times (\text{SP value of component 1})^2 + (\text{volume fraction of component 2}) \times (\text{SP value of component 2})^2 + \cdots$$

The water-soluble compound used in the present invention may be a low molecular weight compound or a high molecular weight compound. The low molecular weight compound preferably has a molecular weight of less than 1,000, and the high molecular weight compound preferably has a weight average molecular weight of 1,000 or more, and more

preferably has a weight average molecular weight of 1,000 or more and 1,000,000 or less.

Values of number average molecular weight (Mn) and weight average molecular weight (Mw) of the high molecular weight compound in the present invention are measured by gel permeation chromatography (GPC).

The water-soluble compound is not particularly limited, but is preferably a compound having at least one bond selected from the group consisting of an ester bond, a carbonate bond, and a sulfonyl bond, and more preferably a compound having at least one bond selected from the group consisting of a carbonate bond and a sulfonyl bond, from the viewpoint of easy water-dismantlability and compatibility with the 2-cyanoacrylate compound.

Preferable examples of the water-soluble high molecular weight compound include polyacrylic acid, polyacrylate, cellulose acetate, and cellulose acetate butyrate.

Among them, the water-soluble compound is preferably at least one compound selected from the group consisting of ethylene carbonate, dimethyl sulfone, sulfolane, γ-butyrolactone, and propylene carbonate, and more preferably at least one compound selected from the group consisting of ethylene carbonate, dimethyl sulfone, sulfolane, and propylene carbonate, from the viewpoint of easy water-dismantlability and compatibility with the 2-cyanoacrylate compound.

From the viewpoint of versatility, the water-soluble compound is preferably at least one compound selected from the group consisting of ethylene carbonate, γ-butyrolactone, dimethylsulfone, and propylene carbonate.

The water-soluble compound represented by Formula (1), which is used in the adhesive composition of the present invention, may be used alone, or two or more thereof can be used in combination.

A content of the water-soluble compound is preferably 0.5 mass% or more and 50 mass% or less, more preferably 1 mass% or more and 40 mass% or less, yet more preferably 5 mass% or more and 35 mass% or less, and particularly preferably 10 mass% or more and 30 mass% or less, with respect to the total mass of the adhesive composition, from the viewpoint of easy water-dismantlability.

When used in an adhesive composition, the adhesive composition may contain other components than the 2-cyanoacrylate compound represented by Formula (1) and the water-soluble compound.

As other components, a stabilizer, a curing accelerator, a plasticizer, a thickener, particles, a colorant, a perfume, a solvent, a strength improver, and the like, which have been conventionally used by being blended in an adhesive composition containing a 2-cyanoacrylate compound, can be blended in an appropriate amount within a range in which curability, adhesive strength, and the like of the adhesive composition are not impaired, depending on the purpose.

Examples of the stabilizer include (1) aliphatic sulfonic acids such as sulfur dioxide and methanesulfonic acid, aromatic sulfonic acids such as p-toluenesulfonic acid, boron trifluoride complexes such as boron trifluoride methanol and boron trifluoride diethyl ether, anionic polymerization inhibitors such as $HBF_4$ and trialkylborate, and (2) radical polymerization inhibitors such as hydroquinone, hydroquinone monomethyl ether, t-butylcatechol, catechol, and pyrogallol. These stabilizers may be used singly, or two or more thereof may be used in combination.

Any curing accelerator can be used as long as it accelerates the anionic polymerization of the 2-cyanoacrylate-based adhesive composition. Examples of the curing accelerator include polyether compounds, calixarenes, thiacalixarenes, pyrogallolarenes, and onium salts. These curing accelerators may be used singly, or two or more thereof may be used in combination.

Examples of the plasticizer include triethyl acetylcitrate, tributyl acetylcitrate, dimethyl adipate, diethyl adipate, dimethyl sebacate, dimethyl phthalate, diethyl phthalate, dibutyl phthalate, diisodecyl phthalate, dihexyl phthalate, diheptyl phthalate, dioctyl phthalate, bis(2-ethylhexyl)phthalate, diisononyl phthalate, diisotridecyl phthalate, dipentadecyl phthalate, dioctyl terephthalate, diisononyl isophthalate, decyl toluate, bis(2-ethylhexyl)camphorate, 2-ethylhexylcyclohexylcarboxylate, diisobutyl fumarate, diisobutyl maleate, triglyceride caproate, 2-ethylhexyl benzoate, and dipropylene glycol dibenzoate. Among them, at least one compound selected from the group consisting of tributyl acetylcitrate, dimethyl adipate, dimethyl phthalate, 2-ethylhexyl benzoate, and dipropylene glycol dibenzoate is preferable from the viewpoint of good compatibility with the 2-cyanoacrylate compound and high plasticization efficiency. These plasticizers may be used singly, or two or more thereof may be used in combination.

Examples of the thickener include polymethyl methacrylate, a copolymer of methyl methacrylate and an acrylic acid ester, a copolymer of methyl methacrylate and any other methacrylic acid ester, acrylic rubber, polyvinyl acetate, polyvinyl chloride, a polyurethane resin, a polyamide resin, polystyrene, a cellulose ester, polyalkyl-2 cyanoacrylic acid ester, and an ethylene-vinyl acetate copolymer. These thickeners may be used singly, or two or more thereof may be used in combination.

The particles that may be blended in the adhesive composition are for adjusting a thickness of the adhesive layer when the adhesive composition is used.

An average particle diameter of the particles is preferably 10 μm to 200 μm, more preferably 15 μm to 200 μm, and yet more preferably 15 μm to 150 μm.

A material for the particles is not particularly limited as long as it is insoluble in the 2-cyanoacrylate compound to be used and does not cause deterioration such as polymerization. Examples of the material for the particles include thermoplastic resins such as polyethylene, polypropylene, polymethylpentene, acrylic resins, polyvinyl chloride, polytetrafluoroethylene, polyethylene terephthalate, polybutylene terephthalate, polysulfone, and polyphenylene oxide; crosslinked resins such as unsaturated polyester, divinylbenzene polymers, divinylbenzene-styrene copolymers, divinylbenzene-(meth) acrylic acid ester copolymers, and diallyl phthalate polymers; inorganic compounds such as spherical silica, glass beads, and glass fiber; silicone compounds; and organic-inorganic composite particles containing an organic polymer skeleton and a polysiloxane skeleton.

A content of the particles is not particularly limited, but is preferably 0.1 parts by mass to 10 parts by mass, more preferably 1 part by mass to 5 parts by mass, and yet more preferably 1 part by mass to 3 parts by mass when the content of the 2-cyanoacrylate compound is 100 parts by mass. When the content is in the range of 0.1 parts by mass to 10 parts by mass, the influence on the curing speed and the adhesive strength can be reduced.

The average particle diameter of the particles in the present invention is a volume-based average value measured by a laser diffraction particle size distribution measuring apparatus.

The adhesive composition is not particularly limited, and can be used for various applications.

Among them, when the adhesive composition is used as an easily water-dismantlable adhesive composition, the easily water-dismantlable adhesive composition enables easy peeling or removal, with water, of the adhesive composition at an adhered part or the adhesive composition attached to an unnecessary part and cured, and therefore can be suitably used as, for example, an easily water-dismantlable adhesive composition for temporary fixation or an easily water-dismantlable adhesive composition for teaching materials. A cured product obtained by curing the easily water-dismantlable adhesive composition is excellent in easy water-dismantlability, and thus can be easily peeled or removed, in a short time, with hot water or pressurized hot water, and can be peeled or removed, for example, by being immersed in water in a temperature range from normal temperature (15°C to 25°C) to about a temperature of lukewarm water (30°C to 45°C). Therefore, for example, even when the easily water-dismantlable adhesive composition is attached to an unnecessary part such as a finger and cured, it can be easily peeled with water. In addition, since it is possible to peel or remove it with water in a temperature range from normal temperature to about a temperature of lukewarm water, and thus to reduce an amount of heat required for peeling, leading to reduction in cost when used for temporary fixation for industrial uses.

The "dismantling" in the present disclosure does not require that the whole cured product is dissolved or dispersed in a solvent such as water, and it is sufficient that at least a part of the cured product be dissolved or dispersed in the solvent, or swelled, and that adhered members can be peeled from each other.

Here, examples of the temporary fixation for industrial uses include temporary fixation of various electronic materials such as semiconductor wafers or optical materials such as lenses with various jigs such as polishing surface plates.

In addition, an adherend to be adhered by the adhesive composition is not particularly limited, and may be an inorganic compound, an organic compound, or an inorganic-organic composite, and may be made of the same material or different materials. In addition, the adhesive composition can adhere a solid material having any shape.

A method for storing the 2-cyanoacrylate compound represented by Formula (1) and the adhesive composition is not particularly limited, and the 2-cyanoacrylate compound and the adhesive composition may be stored using a known method for storing a cyanoacrylate. For example, storage in anhydrous inert gas, storage in a light-shielding sealed container, and the like are suitably exemplified.

## EXAMPLES

Hereinafter, the present invention will be specifically described based on Examples. Note that the present invention is not limited to these Examples. In addition, in the following description, "parts" and "%" mean "parts by mass" and "mass%", respectively, unless otherwise specified.

The SP value of a block in a thermoplastic elastomer was calculated by the method described above.

(Example 1: Synthesis of 2-[2-(2-methoxyethoxy)ethoxy]ethyl-2-cyanoacrylate)

Cyanoacetic acid (247 g, 2.90 mol), 2-[2-(2-methoxyethoxy)ethoxy]ethanol (also called triethylene glycol monomethyl ether) (530 g, 3.23 mol), p-toluenesulfonic acid monohydrate (27.4 g, 0.144 mol), and toluene (500 mL) were esterified while being dehydrated under reflux, and then the esterified product was neutralized with an aqueous sodium carbonate solution, and distilled to obtain 426 g (yield: 64%) of 2-[2-(2-methoxyethoxy)ethoxy]ethyl-2-cyanoacetate. A boiling point of the obtained compound was 133°C to 135°C/10 Pa, and a $^1$H-NMR spectrum thereof was as follows.

$^1$H-NMR($CD_3Cl$): $\delta$=4.38-4.35(m,2H), 3.76-3.73(m,2H), 3.69-3.64(m,6H), 3.57-3.54(m,2H), 3.52(s,2H), 3.38(s,3H)

2-[2-(2-Methoxyethoxy)ethoxy]ethyl-2-cyanoacetate (400 g, 1.73 mol), paraformaldehyde (50.4 g, 1.68 mol), piperidine (0.43 g, 0.0050 mol), and toluene (250 mL) were condensed while generated water was azeotropically dehydrated under reflux. The condensate was washed with water, diphosphorus pentoxide (1.2 g, 0.0084 mol) and SUMILIZER

MDP-S (phenolic antioxidant, manufactured by Sumitomo Chemical Co., Ltd.) were added to the organic layer, and depolymerization was performed to obtain a fraction having a boiling point of 110°C to 125°C/10 Pa. This fraction was re-distilled to obtain 42 g (yield: 10%) of 2-[2-(2-methoxyethoxy)ethoxy]ethyl-2-cyanoacrylate having a boiling point of 120°C to 122°C/7.5 Pa.

$^1H$ and $^{13}C$-NMR spectra of the obtained 2-[2-(2-methoxyethoxy)ethoxy]ethyl-2-cyanoacrylate are as follows.

$^1H$-NMR($CD_3Cl$): δ=7.08(s,1H), 6.65(s,1H), 4.44-4.42(m,2H), 3.81-3.78(m,2H), 3.71-3.64(m,6H), 3.56-3.54(m,2H), 3.38(s,3H)

$^{13}C$-NMR($CD_3Cl$): δ=160.5, 143.4, 116.6, 114.3, 71.9, 70.8, 70.6, 70.6, 68.5, 65.9, 59.0

In addition, the $^1H$-NMR spectrum of 2-[2-(2-methoxyethoxy)ethoxy]ethyl-2-cyanoacrylate is shown in Fig. 1, and the $^{13}C$-NMR spectrum thereof is shown in Fig. 2.

(Example 2: Synthesis of 2-[2-(2-methoxypropoxy)propoxy]propyl-2-cyanoacrylate (isomer mixture))

Cyanoacetic acid (188 g, 2.21 mol), 2-[2-(2-methoxypropoxy)propoxy]propanol (isomer mixture) (also called tripropylene glycol monomethyl ether) (500 g, 2.42 mol), p-toluenesulfonic acid monohydrate (21.0 g, 0.11 mol), and toluene (500 mL) were esterified while being dehydrated under reflux, and then the esterified product was neutralized with an aqueous sodium carbonate solution, and distilled to obtain 434 g (yield: 72%) of 2-[2-(2-methoxypropoxy)propoxy] propyl-2-cyanoacrylate. A boiling point of the obtained compound was 127°C to 130°C/10 Pa, and a $^1H$-NMR spectrum thereof was as follows.

$^1H$-NMR($CD_3Cl$): δ=5.21-5.09(m,1H), 3.67-3.29(m,13H), 1.28-1.27(m,3H), 1.15-1.12(m,6H)

2-[2-(2-Methoxypropoxy)propoxy]propyl-2-cyanoacrylate (400 g, 1.46 mol), paraformaldehyde (42.6 g, 1.42 mol), piperidine (0.36 g, 0.0042 mol), and toluene (250 mL) were condensed while generated water was azeotropically dehydrated under reflux. The condensate was washed with water, diphosphorus pentoxide (1.2 g, 0.00838 mol) and SUMILIZER MDP-S (manufactured by Sumitomo Chemical Co., Ltd.) were added to the organic layer, and depolymerization was performed to obtain a fraction having a boiling point of 110°C to 125°C/10 Pa. This fraction was re-distilled to obtain 40 g (yield: 10%) of 2-[2-(2-methoxypropoxy)propoxy]propyl-2-cyanoacrylate having a boiling point of 123°C to 125°C/10 Pa.

$^1H$ and $^{13}C$-NMR spectra of the obtained 2-[2-(2-methoxypropoxy)propoxy]propyl-2-cyanoacrylate are as follows.

$^1H$-NMR($CD_3Cl$): δ=7.05(s,1H), 6.62(s,1H), 5.23-5.14(m,1H), 3.67-3.29(m,11H), 1.33-1.31(m,3H), 1.15-1.11(m,6H)

$^{13}C$-NMR($CD_3Cl$): δ=160.0, 143.0, 117.1, 114.5, 76.7-76.6, 75.9-75.6, 75.1-74.9, 73.5-73.1, 71.6-71.2, 59.1, 56.7, 17.3-16.9, 16.5-16.4

In addition, the $^1H$-NMR spectrum of 2-[2-(2-methoxypropoxy)propoxy]propyl-2-cyanoacrylate is shown in Fig. 3, and the $^{13}C$-NMR spectrum thereof is shown in Fig. 4.

(Example 3: Synthesis of 2-[2-(2-ethoxyethoxy)ethoxy]ethyl-2-cyanoacrylate)

Cyanoacetic acid (276 g, 3.24 mol), 2-[2-(2-ethoxyethoxy)ethoxy]ethanol (also called triethylene glycol monoethyl ether) (633 g, 3.55 mol), p-toluenesulfonic acid monohydrate (29.5 g, 0.155 mol), and toluene (500 mL) were esterified while being dehydrated under reflux, and then the esterified product was neutralized with an aqueous sodium carbonate solution, and distilled to obtain 580 g (yield: 73%) of 2-[2-(2-ethoxyethoxy)ethoxy]ethyl-2-cyanoacrylate. Aboiling point of the obtained compound was 128°C to 133°C/10 Pa, and a $^1H$-NMR spectrum thereof was as follows.

$^1H$-NMR($CD_3Cl$): δ=4.38-4.35(m,2H), 3.76-3.73(m,2H), 3.68-3.64(m,6H), 3.61-3.58(m,2H), 3.56-3.50(m,4H), 1.21(t,3H)

2-[2-(2-Ethoxyethoxy)ethoxy]ethyl-2-cyanoacrylate (400 g, 1.63 mol), paraformaldehyde (47.5 g, 1.58 mol), piperidine (0.41 g, 0.0048 mol), and toluene (250 mL) were condensed while generated water was azeotropically dehydrated under reflux. The condensate was washed with water, diphosphorus pentoxide (1.2 g, 0.0084 mol) and SUMILIZER MDP-S (phenolic antioxidant, manufactured by Sumitomo Chemical Co., Ltd.) were added to the organic layer, and depolymerization was performed to obtain a fraction having a boiling point of 110°C to 135°C/10 Pa. This fraction was re-distilled to obtain 32 g (yield: 8%) of 2-[2-(2-ethoxyethoxy)ethoxy]ethyl-2-cyanoacrylate having a boiling point of 120°C to 122°C/7.5 Pa.

$^1H$ and $^{13}C$-NMR spectra of the obtained 2-[2-(2-ethoxyethoxy)ethoxy]ethyl-2-cyanoacrylate are as follows.

$^1H$-NMR($CD_3Cl$): δ=7.07(s,1H), 6.64(s,1H), 4.44-4.42(m,2H), 3.81-3.78(m,2H), 3.71-3.64(m,6H), 3.58-3.55(m,2H), 3.55-3.50(m,2H), 1.21(t,3H)

$^{13}C$-NMR($CD_3Cl$):
δ=160.5,143.3,116.7,114.3,70.8,70.8,70.7,69.8,68.6,66.6,65.9,15.2

In addition, the $^1H$-NMR spectrum of 2-[2-(2-ethoxyethoxy)ethoxy]ethyl-2-cyanoacrylate is shown in Fig. 5, and

the $^{13}$C-NMR spectrum thereof is shown in Fig. 6.

(Example 4: Synthesis of 2-[2-(2-butoxyethoxy)ethoxy]ethyl-2-cyanoacrylate)

Cyanoacetic acid (193 g, 2.27 mol), 2-[2-(2-butoxyethoxy)ethoxy]ethanol (also called triethylene glycol monobutyl ether) (499 g, 2.42 mol), p-toluenesulfonic acid monohydrate (21.6 g, 0.114 mol), and toluene (500 mL) were esterified while being dehydrated under reflux, and then the esterified product was neutralized with an aqueous sodium carbonate solution, and distilled to obtain 460 g (yield: 74%) of 2-[2-(2-butoxyethoxy)ethoxy]ethyl-2-cyanoacrylate. A boiling point of the obtained compound was 134°C to 138°C/7.5 Pa, and a $^{1}$H-NMR spectrum thereof was as follows.

$^{1}$H-NMR($CD_3Cl$): δ=4.38-4.35(m,2H), 3.76-3.73(m,2H), 3.67-3.63(m,6H), 3.60-3.57(m,2H), 3.51(s,2H), 3.46(t,2H), 1.60-1.53(m,2H), 1.41-1.32(m,2H), 0.92(t,3H)

2-[2-(2-Butoxyethoxy)ethoxy]ethyl-2-cyanoacrylate (400 g, 1.46 mol), paraformaldehyde (42.5 g, 1.42 mol), piperidine (0.37 g, 0.0043 mol), and toluene (250 mL) were condensed while generated water was azeotropically dehydrated under reflux. The condensate was washed with water, diphosphorus pentoxide (1.2 g, 0.0084 mol) and SUMILIZER MDP-S (phenolic antioxidant, manufactured by Sumitomo Chemical Co., Ltd.) were added to the organic layer, and depolymerization was performed to obtain a fraction having a boiling point of 120°C to 140°C/10 Pa. This fraction was re-distilled to obtain 25 g (yield: 6%) of 2-[2-(2-butoxyethoxy)ethoxy]ethyl-2-cyanoacrylate having a boiling point of 127°C to 136°C/10 Pa.

$^{1}$H and $^{13}$C-NMR spectra of the obtained 2-[2-(2-butoxyethoxy)ethoxy]ethyl-2-cyanoacrylate are as follows.

$^{1}$H-NMR($CD_3Cl$): δ=7.07(s,1H), 6.64(s,1H), 4.44-4.42(m,2H), 3.81-3.78(m,2H), 3.70-3.63(m,6H), 3.60-3.57(m,2H), 3.46(t,2H), 1.60-1.53(m,2H), 1.41-1.31(m,2H), 0.91(t,3H)

$^{13}$C-NMR($CD_3Cl$): δ=160.5, 143.3, 116.7, 114.3, 71.2, 70.8, 70.7, 70.7, 70.1, 68.6, 65.9, 31.7, 19.3, 13.9

In addition, the $^{1}$H-NMR spectrum of 2-[2-(2-butoxyethoxy)ethoxy]ethyl-2-cyanoacrylate is shown in Fig. 7, and the $^{13}$C-NMR spectrum thereof is shown in Fig. 8.

(Example 5: Synthesis of 2-[2-(2-butoxypropoxy)propoxy]propyl-2-cyanoacrylate)

Cyanoacetic acid (193 g, 2.27 mol), 2-[2-(2-butoxypropoxy)propoxy]propanol (also called tripropylene glycol monobutyl ether) (622 g, 2.50 mol), p-toluenesulfonic acid monohydrate (27.4 g, 0.144 mol), and toluene (500 mL) were esterified while being dehydrated under reflux, and then the esterified product was neutralized with an aqueous sodium carbonate solution, and distilled to obtain 511 g (yield: 71%) of 2-[2-(2-butoxypropoxy)propoxy]propyl-2-cyanoacrylate. A boiling point of the obtained compound was 130°C to 132°C/10 Pa, and a $^{1}$H-NMR spectrum thereof was as follows.

$^{1}$H-NMR($CD_3Cl$): δ=5.19-5.09(m,1H), 3.65-3.30(m,12H), 1.59-1.52(m,2H), 1.41-1.32(m,2H), 1.28-1.26(m,3H), 1.15-1.11(m,6H), 0.92(t,3H)

2-[2-(2-Butoxypropoxy)propoxy]propyl-2-cyanoacrylate (400 g, 1.27 mol), paraformaldehyde (37.0 g, 1.23 mol), piperidine (0.32 g, 0.0038 mol), and toluene (250 mL) were condensed while generated water was azeotropically dehydrated under reflux. The condensate was washed with water, diphosphorus pentoxide (1.2 g, 0.0084 mol) and SUMILIZER MDP-S (phenolic antioxidant, manufactured by Sumitomo Chemical Co., Ltd.) were added to the organic layer, and depolymerization was performed to obtain a fraction having a boiling point of 110°C to 130°C/10 Pa. This fraction was re-distilled to obtain 33 g (yield: 8%) of 2-[2-(2-butoxypropoxy)propoxy]propyl-2-cyanoacrylate having a boiling point of 118°C to 120°C/15 Pa.

$^{1}$H and $^{13}$C-NMR spectra of the obtained 2-[2-(2-butoxypropoxy)propoxy]propyl-2-cyanoacrylate are as follows.

$^{1}$H-NMR($CD_3Cl$): δ=7.04(s,1H), 6.60(s,1H), 5.22-5.14(m,1H), 3.69-3.29(m,10H), 1.58-1.51(m,2H), 1.41-1.30(m,5H), 1.14-1.11(m,6H), 0.91(t,3H)

$^{13}$C-NMR($CD_3Cl$): δ=160.0, 142.9, 117.2, 114.5, 75.9-74.7, 73.6-73.1, 71.6-71.2, 69.0, 32.2, 31.8, 19.3, 17.3-17.2, 16

In addition, the $^{1}$H-NMR spectrum of 2-[2-(2-butoxypropoxy)propoxy]propyl-2-cyanoacrylate is shown in Fig. 9, and the $^{13}$C-NMR spectrum thereof is shown in Fig. 10.

The 2-cyanoacrylate compound of the present invention can be suitably used as a cured component of an adhesive composition. The 2-cyanoacrylate compound can be used, for example, as a so-called instant adhesive in a wide range of products and technical fields such as general household uses, teaching materials, building materials, and medical fields as well as various industrial fields.

In addition, the adhesive composition containing a 2-cyanoacrylate compound of the present invention can be suitably used for adhesion not only between the same kind of materials to be adhered to each other but also between different kinds of materials to be adhered to each other (for example, between a metal and a resin).

The disclosure of Japanese Patent Application No. 2019-146572 filed on August 8, 2019 is incorporated herein by reference in its entirety.

All documents, patent applications, and technical standards described herein are incorporated herein by reference to the same extent as if each document, patent application, and technical standard are specifically and individually indicated to be incorporated by reference.

## Claims

1. A 2-cyanoacrylate compound represented by the following Formula (1):

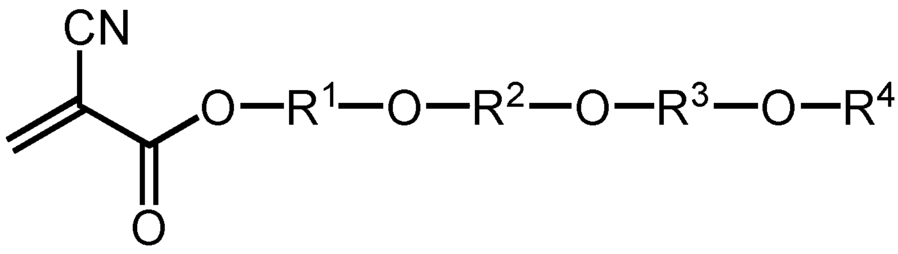

(1)

wherein $R^1$ represents a divalent linking group having 1 to 20 carbon atoms, $R^2$ and $R^3$ each independently represent an alkylene group having 2 to 4 carbon atoms, and $R^4$ represents an alkyl group having 1 to 20 carbon atoms.

2. The 2-cyanoacrylate compound according to claim 1, wherein $R^1$ is an alkylene group having 1 to 20 carbon atoms.

3. The 2-cyanoacrylate compound according to claim 1 or 2, wherein $R^1$ is an alkylene group having 1 to 8 carbon atoms.

4. The 2-cyanoacrylate compound according to any one of claims 1 to 3, wherein $R^2$ and $R^3$ are each independently an ethylene group, a 1,2-propylene group, or a 1,3-propylene group.

5. The 2-cyanoacrylate compound according to any one of claims 1 to 4, wherein $R^4$ is an alkyl group having 1 to 6 carbon atoms.

6. The 2-cyanoacrylate compound according to any one of claims 1 to 5, wherein $R^4$ is an alkyl group having 1 to 4 carbon atoms.

7. An adhesive composition comprising the 2-cyanoacrylate compound according to any one of claims 1 to 6.

**INTERNATIONAL SEARCH REPORT**

International application No. PCT/JP2020/029905

A. CLASSIFICATION OF SUBJECT MATTER
C07C 255/23(2006.01)i; C09J 4/04(2006.01)i
FI: C07C255/23 CSP; C09J4/04
According to International Patent Classification (IPC) or to both national classification and IPC

B. FIELDS SEARCHED
Minimum documentation searched (classification system followed by classification symbols)
C07C255/23; C09J4/04; C08F20/00-22/40

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 3-12647 A (FUJI PHOTO FILM CO., LTD.) 21.01.1991 (1991-01-21) page 6, lower left column, lines 3-4, compound example 21 (C-21) | 1-6<br>7 |
| X | JP 2014-532488 A (INSTITUTE OF PHARMACOLOGY AND TOXICOLOGY ACADEMY OF MILITARY MEDICAL SCIENCES P.L.A. CHINA) 08.12.2014 (2014-12-08) claim 1, table 2, J13 | 1-7 |
| X | WO 2018/067615 A1 (SIGILON THERAPEUTICS, INC.) 12.04.2018 (2018-04-12) fig. 2C, 3C, 3HH, 5C, 5T | 1-6 |
| A | JP 63-60961 A (SHIN-ETSU CHEMICAL CO., LTD.) 17.03.1988 (1988-03-17) claim 1, page 3, upper right column, lines 4-8 | 1-7 |
| A | JP 56-135455 A (TOA-GOSEI CHEMICAL INDUSTRY CO., LTD.) 22.10.1981 (1981-10-22) entire text | 1-7 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 September 2020 (14.09.2020) | 29 September 2020 (29.09.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/029905

| Patent Documents referred in the Report | Publication Date | Patent Family | Publication Date |
|---|---|---|---|
| JP 3-12647 A | 21 Jan. 1991 | EP 401826 A1<br>page 11, lines 36-44, (C-21)<br>US 5153115 A | |
| JP 2014-532488 A | 08 Dec. 2014 | US 2014/0369952 A1<br>claim 1, table 2, J13<br>WO 2013/064059 A1<br>EP 2792372 A1<br>CN 103083718 A | |
| WO 2018/067615 A1 | 12 Apr. 2018 | JP 2019-529579 A<br>fig. 2C, 3C, 3HH, 5C, 5T<br>CN 110036008 A<br>KR 10-2019-0092374 A | |
| JP 63-60961 A | 17 Mar. 1988 | (Family: none) | |
| JP 56-135455 A | 22 Oct. 1981 | US 4364876 A<br>entire text<br>GB 2073183 A<br>DE 3111974 A<br>FR 2479208 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017135184 A
- JP 2017514796 A
- JP H8283225 A
- JP 2000073015 A
- JP H8188747 A
- JP 2019146572 A


### Non-patent literature cited in the description


- *Polymer Engineering and Science,* 1974, vol. 14 (2), 147